# EUROPEAN PATENT APPLICATION

(11) **EP 2 659 928 A1**
(43) Date of publication of application: **06.11.2013**
(21) Application number: 11853456.9
(22) Date of filing: 26.01.2011
(51) Int. Cl.: A61M 16/04, A61M 16/00

(54) **TRACHEAL TUBE WITH FLAT MOUTH AND SIDE OPENING AND GUIDE CORE**

(30) Priority: 29.12.2010 CN 201010620049
(71) Applicant: Chen, Zhiyang, Shanghai 201104 (CN); Chen, Ruiguang, Shanghai 201104 (CN); Liu, Jin, Shanghai 201104 (CN)
(72) Inventor: Chen, Zhiyang, Shanghai 201104 (CN); Chen, Ruiguang, Shanghai 201104 (CN); Liu, Jin, Shanghai 201104 (CN)
(74) Representative: Petraz, Gilberto Luigi
(86) International application number: PCT/CN2011/000123
(87) International publication number: WO 2012/088729

(57) **Abstract**

A tracheal tube with a flat mouth and a side opening for trachea intubation is provided. The front end of the tracheal tube is a flat mouth with circular cross section. The tracheal tube has a side opening (2) and an air pocket (3). The inner diameter of the tracheal tube is 5 to 10 mm. A guide core for use with the tracheal tube is composed of a hemisphere, a front cylinder, a truncated cone and a rear cylinder. The hemisphere, front cylinder, truncated cone and rear cylinder of the guide core reach the trachea through glottis in turn during intubation, and then the tracheal tube reaches the trachea. Said tracheal tube and guide core avoid blocking the doctor' view, which greatly improving success ratio of intubation, and avoiding injury to the throat by the tracheal tube.

## Description

### BACKGROUND OF THE INVENTION

### 1. Field of the Invention

This invention involves a tracheal tube for clinical anesthesia, emergency care and intensive care. The front end of the tracheal tube is a flat mouth. The inner diameter of the tracheal tube is 5 to 10 mm. The tracheal tube has a side opening and an air pocket. A guide core is used to guide the tracheal tube into trachea. The front end of the guide core is a hemisphere having a diameter of 3 to 5 mm. The front end of the guide core is followed by a front cylinder with a length of 30 to 50 mm and a diameter equal to the diameter of the hemisphere in front. The front cylinder is followed by a truncated cone, the diameter of the front cross section of the truncated cone being equal to the diameter of the front cylinder. The diameter of the rear cross section is equal to the diameter of the following rear cylinder, and the truncated cone is 10 to 30 mm in length. The truncated cone is followed by the rear cylinder, which is 5 to 9 mm in diameter and 50 to 70 cm in length.

### 2. Description of Prior Art

Presently, a tracheal tube with a slanted mouth at the front and a side opening is commonly used worldwide in clinical settings such as anesthesia. The slanted mouth at the front of a tracheal tube is meant to adapt to the shape of the glottis (i.e., "1" shaped). This is to make it easier to slip the tracheal tube through the glottis. However, because the slanted area at the front of the tracheal tube is relatively large, it can easily get stuck at the glottis, epiglottis and larynx to make intubation more difficult. Damage to the throat by the slanted mouth can happen easily after repeated intubation attempts with brute force. This can lead to laryngeal edema, respiratory distress and even patient asphyxia. As a matter of fact, this is the most common cause of patient death encountered during intubation. When intubation becomes difficult, regardless of whether a bronchoscope or a bougie is used to assist intubation, after the successful insertion of a bronchoscope or bougie into the trachea, it is often difficult to push the tracheal tube further down the windpipe. This is because the tracheal tube can easily get stuck when its slanted mouth encounters the piriform sinus, epiglottis or vocal cords if the leading edge of the bronchoscope or bougie forms an angle with the insertion end of the tracheal tube. When brute force is used to push the tube down the trachea, it can cause damage to the vocal cords, epiglottis and larynx. The bronchoscope may also be damaged. Intubation becomes very difficult. To overcome this problem, a tracheal tube with a flat mouth, a side oponing and a guide core was designed. The leading edge of the tracheal tube has a circularly shaped flat mouth. The inner diameter at the opening is 5 to 10 mm. In addition, there is a side opening to enhance ventilation. The tracheal tube is equipped with a guide core for entering the trachea. The front of the guide core is a hemisphere with a diameter of 3 to 5 mm. It is followed by a front cylinder, which is 30 to 50 mm in length and has a diameter equal to the diameter of the hemisphere. It is then followed by a truncated cone having a length of 10 to 30 mm. The truncated cone is followed by a rear cylinder, which is 5 to 9 mm in diameter and 50 to 70 cm in length. The advantage of this guide core is that its front end is hemispherical in shape, which makes it easier to pass through the glottis to minimize damage to the vocal cords and the larynx. The view of the physician performing intubation under the laryngoscope is not obstructed by the tracheal tube. The leading hemisphere and the front cylinder of the guide core enter the glottis first, followed by the gradually thickened truncated cone and rear cylinder. This is followed by the insertion of the tracheal tube, which has an inner diameter slightly larger than the diameter of the rear cylinder of the guide core. Regardless of whether intubation is routine or difficult, this tracheal tube with a flat mouth, side opening and guide core can provide a clear view for the physician performing intubation and avoids damage to the vocal cords, epiglottis and piriform sinus in order to significantly improve the success rate of intubation.

### SUMMARY OF THE INVENTION

Presently, a tracheal tube with a slanted mouth at the front is commonly used clinically. Once it gets stuck when passing through the glottis, damage to the vocal cords, epiglottis and piriform sinus can occur quite easily. A tracheal tube with a flat mouth, a side opening and a guide core was designed. The front end is a circularly shaped flat mouth. The guide core passes through the glottis in the following order; i.e., front hemisphere, front cylinder, truncated cone and rear cylinder. The tracheal tube is then inserted into the trachea along the guide core. Afterward, the guide core can be withdrawn. Damage to the larynx can be avoided by using the tracheal tube with a flat mouth and side opening. When intubation is assisted by using a bronchoscope or bougie, choosing the tracheal tube with a flat mouth, a side opening and a suitable diameter can make tube delivery even easier. As a result, there will be a significant increase in the success rate of intubation.

In the present invention, the technical solution adopted for solving the technical issues can be summarized as follows: As far as the parts of the tracheal tube are concerned, there is difference when compared to the ordinary tracheal tube in use today. The only difference is that the tracheal tube of the present invention is designed to be equipped with a circularly shaped flat mouth at the front. The inner diameter of the tracheal tube of the present invention is 5 - 10 mm. In addition, the tracheal tube of the present invention has a side opening, which is elliptical in shape to enhance ventilation. A guide core for use with the tracheal tube is also provided. The front end of the guide core is a hemisphere having a diameter of 3 to 5 mm. The front end of the guide core is followed by a front cylinder with a length of 30 to 50 mm and a diameter equal to the diameter of the hemisphere in front. The front cylinder is followed by a truncated cone. The diameter of the front cross section of the truncated cone is equal to the diameter of the front cylinder and the diameter of the rear cross section is equal to the diameter of the following rear cylinder, and the truncated cone is 10 to 30 mm in length. The truncated cone is followed by the rear cylinder, which is 5 to 9 mm in diameter and 50 to 70 cm in length.

The advantages of this invention can be summarized as follows. There is minimal contact between the tracheal tube and the larynx. Relatively smaller components, such as the leading hemisphere and front cylinder, pass through the glottis into the trachea first. Because of this the field of view of the physician performing intubation is not blocked by the tracheal tube. The small components are followed by insertion of gradually thickened components such as the truncated cone, rear cylinder and tracheal tube with a flat mouth and side opening, which are introduced into the trachea through the glottis. There will be a significant increase in the success rate of intubation. Damage done to the vocal cords, epiglottis and piriform sinus by a tracheal tube can be avoided. In comparison to an ordinary tracheal tube with a slanted mouth, unless the slanted mouth is directly aligned with the glottis fissure, damage to the vocal cords and larynx can occur extremely easily when brute force is used by the physician performing intubation if obstruction is encountered during intubation. In addition, intubation is made more difficult with existing tracheal tubes.

### BRIEF DESCRIPTION OF THE DRAWINGS

The following is a more detailed description of this invention in conjunction with the drawing and preferred embodiments.

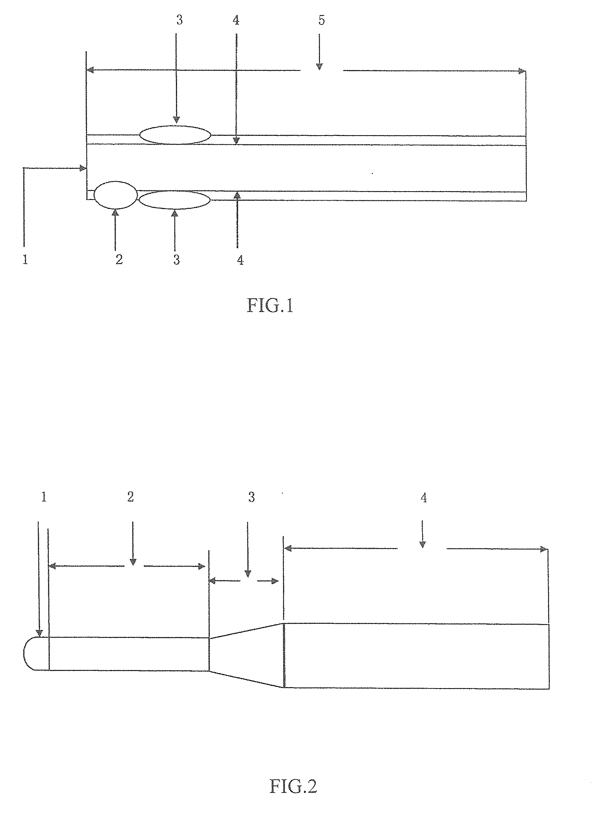

Figures 1 and 2 are schematic diagrams of this invention.

Figure 1 Tracheal tube with flat mouth and side opening, wherein 1 - flat mouth; 2 - side opening; 3 - air-pocket cuff; 4 - inner diameter (5.5 to 10 mm); 5 - tube body.

Figure 2 Guide Core, wherein 1 - hemisphere (3 to 5 mm in diameter); 2 - front cylinder (30 to 50 mm in length and diameter equal to the diameter of the hemisphere in front); 3 - truncated cone (diameter of the front cross section is equal to the diameter of the front cylinder, while the diameter of the rear cross section is equal to the diameter of the rear cylinder, and its length is equal to 10 to 30 mm); 4 - rear cylinder (5 to 9 mm in diameter and 50 to 70 cm in length).

### DETAILED DESCRIPTION OF THE INVENTION

Figure 1 shows a tracheal tube with a flat mouth and a side opening. The front end of the tracheal tube is a flat mouth with circular cross section. The side opening is next to the flat mouth. An air pocket is behind the opening, which can be filled with air. The inner diameter of the flat mouth is 5 to 10 mm. Figure 2 shows the guide core. The front end of the guide core is a hemisphere with a diameter of 3 to 5 mm. The front end of the guide core is followed by a front cylinder with a length of 30 to 50 mm and a diameter equal to the diameter of the hemisphere in front. The front cylinder is followed by a truncated cone. The diameter of the front cross section of the truncated cone is equal to the diameter of the front cylinder and the diameter of its rear cross section is equal to the diameter of the rear cylinder, and the length of the truncated cone is equal to 10 to 30 mm. The truncated cone is followed by a rear cylinder with a diameter equal to 5 to 9 mm and a length of 50 to 70 cm.

The inner diameter the tracheal tube with a flat mouth and a side opening is 5 to 10 mm. A tracheal tube with a suitable inner diameter can be chosen by the anesthesiologist, based on the patient's requirement. The diameter of the rear cylinder of the guide core is 1 to 2 mm smaller than the inner diameter of the tracheal tube. In patients for whom difficulties are anticipated in intubation, the guide core may be inserted into the trachea first, by introducing the front hemisphere, front cylinder, truncated cone and rear cylinder through the glottis. Then, the tracheal tube with a flat mouth and a side opening can be inserted along the guide core. The guide core can then be withdrawn. The guide core may be inserted into the tracheal tube with a flat mouth and a side opening up to the junction of the rear cylinder and the truncated cone. The physician performing intubation may hold the junction between the end of the tracheal tube and its guide core to allow the leading hemisphere, front cylinder, truncated cone and rear cylinder to pass through the glottis in that particular order. After the air pocket enters the glottis, the guide core is withdrawn gradually. After the tracheal tube is inserted to the desired depth, the guide core can be totally withdrawn and the air pocket may be inflated. After confirming that the tracheal tube is located in the trachea, intubation is completed.

## Claims

1. A tracheal tube with a flat mouth and a side opening, equipped with a circularly shaped flat mouth, a side opening and an air pocket; and a matching guide core composing of a hemisphere, a front cylinder, a truncated cone and a rear cylinder.

2. The tracheal tube with a flat mouth and a side opening as claimed in Claim 1, **characterized in that**: the front end of the tracheal tube is a flat mouth with circular cross section, the tracheal tube has a side opening and an air pocket, and the inner diameter of the tracheal tube is 5 to 10 mm.

3. The guide core as claimed in Claim 1, composing of a hemisphere, a front cylinder, a truncated cone and a rear cylinder, and **characterized in that**:: the front hemisphere has a diameter of 3 to 5 mm; the front cylinder has a length of 30 to 50 mm and a diameter equal to the diameter of the hemisphere in front; the truncated cone has a length of 10 to 30 mm, and the diameter of the front cross section of the truncated cone is equal to the diameter of the front cylinder, while the diameter of the rear cross section of the truncated cone is equal to the diameter of the rear cylinder; and the rear cylinder has a diameter of 5 - 9 mm and a length of 50 - 70 cm.
